Europäisches Patentamt

European Patent Office

Office européen des brevets

(19)

(11) Publication number: **0 236 104**
**A2**

# EUROPEAN PATENT APPLICATION

(12)

(21) Application number: **87301788.3**

(22) Date of filing: **02.03.87**

(51) Int. Cl.⁴: **A 61 F 13/02**

(30) Priority: **03.03.86 GB 8605214**

(43) Date of publication of application:
**09.09.87 Bulletin 87/37**

(84) Designated Contracting States: **DE FR GB IT**

(71) Applicant: **COURTAULDS PLC**
**18, Hanover Square**
**London W1A 2BB (GB)**

(72) Inventor: **Burrow, Thomas Richard**
**207 Albany Road**
**Coventry West Midlands (US)**

(74) Representative: **Hale, Stephen Geoffrey et al**
**J.Y. & G.W. Johnson Furnival House 14/18 High Holborn**
**London WC1V 6DE (GB)**

(54) **Wound dressing.**

(57) A dressing for wounds comprises a waterproof, water-vapour-permeable polymer film (12, 22) secured within a frame (11, 21) of water-vapour-permeable polymer foam, the water-vapour permeability being at least 300 grams per square metre per 24 hours at 40°/80 per cent relative humidity. One face of the foam frame (11, 21) is coated with a pressure-sensitive adhesive which secures the film (12, 22) to the frame (11, 21) and which extends outwards beyond the film (12, 22) to provide an adhesive surface for securing the dressing to the skin around the wound. The waterproof, water-vapour-permeable polymer film may act as the facing layer (12) of the dressing in contact with the wound or an absorbent material may be secured within the foam frame (21) over the film (22) by means of the adhesive to form a wound-facing layer (26).

FIG.1

**Description**

## Wound Dressing

This invention relates to wound dressings, particularly for wet wounds such as ulcers and pressure sores.

A type of wound dressing which has been widely used in recent years comprises a waterproof, water-vapour-permeable, adhesive-coated polyurethane film, as described for example in GB-A-1280631 and EP-A-90564. This type of dressing has the advantages that it is self-adhesive, water-vapour-permeable to prevent maceration of the skin, transparent to allow observation of the wound, flexible and conformable to the body surface and that it forms a barrier to water-borne and air-borne chemicals and organisms. However, the adhesive coating of the dressing sticks to the wound margins and may cause damage to the wound margins when the dressing is being applied or removed. The dressing is also difficult to handle as the flexible film readily folds back on itself and the adhesive is highly agressive when it contacts areas coated with the same adhesive.

EP-A-122085 describes a dressing comprising a conformable film having slits therethrough, which may have a layer of adhesive along two opposing edges whereby the dressing can be adhered to the skin. EP-A-94755 describes a dressing in the form of a single layer of porous non-woven polyester having a pressure-sensitive adhesive adhesive in marginal areas.

It is desirable to provide an improved dressing for wounds.

A wound dressing according to the invention comprises a waterproof polymer film having a water-vapour permeability of at least 300 grams per square metre per 24 hours at 40°C/80 per cent relative humidity (RH), and is characterised in that the film is secured within a frame of polymer foam, the foam frame being coated on one face with a pressure-sensitive adhesive which secures the film to the frame and which extends outwards beyond the film to provide an adhesive surface for securing the dressing to skin around the wound, the water vapour permeability of the adhesive-coated foam frame being at least 300 grams per square metre per 24 hours at 40°C/80 per cent RH.

The polymer film is preferably transparent and preferably has a low modulus, for example less than 3450 kPa (500 psi) at 300 per cent extension. One type of preferred film is a polyurethane, for example a polyurethane such as "Estane 5702" (Trademark) described in GB-A-1280631. The film is not coated with an adhesive. The film may be a single layer or may have more than one layer, for example a film may be composed of two or more layers of the same polymer coated on one another to ensure that any imperfections are covered so that the film provides an effective barrier to micro-organisms. The water-vapour permeability of the film is preferably at least 500 grams per square metre per 24 hours at 40°C/80 per cent RH.

The foam is preferably a flexible polyurethane foam and may for example be 0.4 to 2.0 mm thick. The pressure-sensitive adhesive is preferably acrylic-based, although other polymers can be used. The adhesive should adhere more strongly to the foam and film than to skin, so that the dressing can be cleanly removed from the skin after use. The water-vapour permeability of the adhesive-coated foam is preferably at least 500 grams per square metre per 24 hours at 40°C/80 per cent RH. The film and adhesive-coated foam are preferably oxygen-permeable to allow the wound and skin to breathe; in practice film and foam having the required water-vapour permeability are generally also oxygen-permeable.

The width of the foam frame should be sufficient to allow good adhesion of the film to the foam and of the dressing to the skin and is preferably 12 to 40 mm. The opening of the frame is of the size and shape required so that the frame surrounds the wound without touching the wound margins. It may for example be circular, elliptical, square or rectangular. The dressings can be supplied to hospitals in a range of sizes. The frame can be cut from a sheet of foam. The foam is preferably coated with the pressure-sensitive adhesive before the frame is cut out. The adhesive can be covered by a release sheet during the cutting operation if desired. A suitable polyurethane foam sheet coated with pressure-sensitive adhesive is sold under the Tradename "Medifix MPU 15". Dressings of different sizes can be cut from the sheet of foam by cutting in concentric circles separated by the desired width of frame, thus reducing wastage of the foam material.

A water-vapour-permeable polymer film of appropriate size and shape is then positioned across the opening in the frame and is secured by pressing it against the adhesive. The film preferably overlaps on to the frame by 3-10 mm to provide adequate adhesion of the film to the foam. The area of adhesive on the frame extending beyond the polymer film for adhesion to the skin around the wound is preferably 10 to 30 mm wide.

In the simplest form of dressing according to the invention the waterproof, water-vapour-permeable polymer film acts as the facing layer of the dressing in contact with the wound.

The dressing of the invention has the advantage that adhesive does not contact the wound and thus cannot stick to the wound margins, if a dressing of adequate size is used. It also has the advantage that the poly urethane foam forms a frame which, although flexible, is stiffer than the film and facilitates handling and positioning of the dressing.

When treating some types of wound a problem is encountered with the accumulation of liquid exudate from the wound under the film, which can lead to the formation of a pool of liquid. This makes possible the creation of a pathway for micro-organisms between the adhesive and the skin as the liquid applies a delaminating force to the adhesive bond. The movement of the liquid may also cause discomfort to the patient and may damage newly-forming tissue. It is desirable to provide an improved dressing which overcomes this problem.

In a preferred type of dressing according to the invention an absorbent material is secured within the foam frame over the film by means of the adhesive so as to form a wound-facing layer. The absorbent material is preferably used in the form of a coherent layer. Such a layer can be cut to a size larger than the water-vapour-permeable polymer film but smaller than the outer diameter of the foam frame. The absorbent material can be positioned across the opening of the frame after the polymer film has been secured so that it overlaps the polymer film, for example by 3 to 10 mm. The absorbent layer is secured by pressing it against the adhesive on the foam frame while still leaving an area of adhesive outside the absorbent material for securing the dressing to the skin.

The absorbent material absorbs liquid exuding from the wound and preferably forms a gel in contact with the wound exudate. The gel formed should preferably be transparent and impermeable to micro-organisms and should have a viscosity which substantially prevents it from moving. The absorbent material can be in the form of a film or a fibrous material, for example a non-woven fabric. One preferred absorbent material is an alginate, for example calcium alginate. A fabric of calcium alginate fibres is particularly preferred since the fibres present a greater surface area for contact with the wound exudate, allowing the ready formation of a transparent gel. The absorbent material can alternatively be formed of insoluble absorbent fibres, for example cellulosic fibres, although this loses the advantage of the transparency of the dressing.

The dressing is preferably packaged with a sheet of release material covering the adhesive. The release sheet preferably extends over the whole dressing and beyond at least part of the edge thereof for ease of removal. The release sheet also protects the wound-facing surface.

The invention will now be described by way of example with reference to the accompanying drawings of which:-

Figure 1 is a plan view of one type of dressing according to the invention,

Figure 2 is a plan view of another type of dressing according to the invention and

Figure 3 is a cross-section on the line A.......A' of Figure 2.

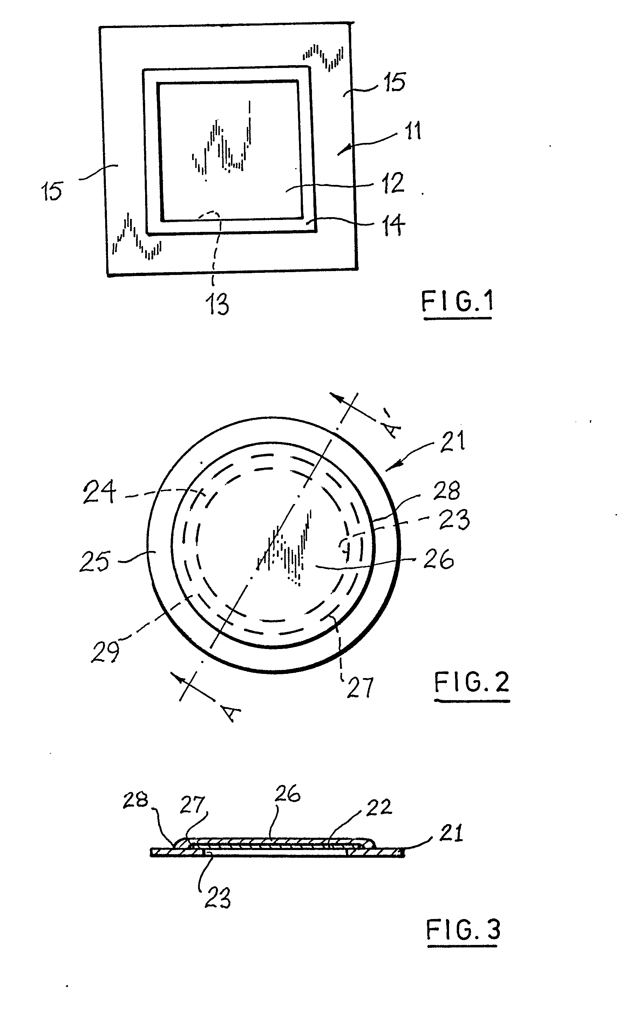

The dressing of Figure 1 comprises a foam frame 11 and a transparent water-vapour-permeable polymer film 12 and is viewed from its wound-facing surface. The visible surface of the foam frame 11 is coated with pressure-sensitive adhesive. The polymer film 12 overlaps the inner edge 13 of the foam frame to form an area 14 in which the polymer film 12 is secured to the foam frame 11 by the adhesive. In the outer area 15 of the foam frame the adhesive remains exposed for adhesion of the dresssing to the skin around the wound.

The dressing of Figures 2 and 3 comprises an adhesive-coated foam frame 21, a transparent water-vapour-permeable polymer film 22 and a gel-forming absorbent layer 26. The polymer film 22 extends across the opening of the foam frame 21 and is bonded to the foam frame in the region 24 between the inner edge 23 of the foam frame and the outer edge 27 of the polymer film. The absorbent layer 26 covers the polymer film 22 when viewed from the wound-facing surface as in Figure 2 and overlaps the outer edge 27 of the polymer film. The absorbent layer 26 is bonded by the adhesive to the foam frame 21 in the region 29 between the outer edge 27 of the polymer film and the outer edge 28 of the absorbent layer. The adhesive coating on the outer area 25 of the foam frame remains free for securing the dressing to the skin around the wound.

The invention is illustrated by the following Examples:-

Example 1

A 0.8 mm thick polyurethane foam sheet coated with pressure-sensitive adhesive and having a water-vapour permeability of 1000 grams per square metre per 24 hours at 40°C/80 per cent RH (Medifix MPU 15) was cut to form a circular frame having internal and external diameters of 70 and 120 mm respectively. A waterproof transparent polyurethane film having a water-vapour permeability of about 1500 grams per square metre per 24 hours at 40°C/80 per cent RH was cut to form a circle of diameter 82 mm and was positioned across the foam frame so that it overlapped the inner edge of the foam frame by about 6 mm in all directions. The polyurethane film was then pressed against the adhesive surface of the foam frame to secure it to the frame. A sheet of release paper was applied over the whole wound-facing surface of the dressing to protect the adhesive which remained exposed at the outer area of the foam frame.

Example 2

A dressing comprising a polyurethane film secured within a foam frame was prepared as described in Example 1. A non-woven web of calcium alginate fibres of weight 100 grams per square metre was cut to form a circle of outside diameter 94 mm and was positioned against the adhesive surface of the foam frame so that it overlapped the polyurethane film by about 6 mm in all directions. The absorbent layer was then pressed against the foam frame to secure it. The resulting dressing was covered with a release sheet as described in Example 1.

**Claims**

1. A dressing for a wound, comprising a waterproof polymer film having a water-vapour permeability of at least 300 grams per square metre per 24 hours at 40°C/80 per cent relative humidity, characterised in that the film (12, 22) is secured within a frame (11, 21) of polymer foam, the foam frame (11, 21) being coated on one face with a pressure-sensitive adhesive which secures the film (12, 22) to the frame (11, 21) and which extends outwards beyond the film (12, 22) to provide an adhesive surface for securing the dressing to skin around the wound, the water-vapour permeability of the adhesive-coated foam frame being at least 300 grams per square metre per 24 hours at 40°C/80 per cent relative humidity.

2. A dressing according to claim 1, characterised in that the film (12, 22) is a polyurethane film having a water-vapour permeability of at least 500 grams per square metre per 24 hours at 40°C/80 per cent relative humidity.

3, A dressing according to claim 1 or claim 2, characterised in that the film (12, 22) comprises a plurality of layers of the same waterproof, water-vapour-permeable polymer.

4. A dressing according to any of claims 1 to 3, characterised in that the frame (11, 21) is formed of flexible polyurethane foam.

5. A dressing according to any of claims 1 to 4, characterised in that the foam frame (11, 21) is 0.4 to 2.0 mm thick.

6. A dressing according to any of claims 1 to 5, characterised in that the film (12) forms the facing layer of the dressing so that in use of the dressing the film is in contact with the wound.

7. A dressing according to any of claims 1 to 5, characterised in that an absorbent material is secured within the frame (21) over the film (22) by means of the pressure-sensitive adhesive so as to form a facing layer (26) so that in use of the dressing the absorbent material is in contact with the wound.

8. A dressing according to claim 7, characterised in that the absorbent material is capable of forming a gel in contact with wound exudate.

9. A dressing according to claim 7 or claim 8, characterised in that the absorbent material is an alginate.

10. A dressing according to claim 9, characterised in that the absorbent layer (26) is a fabric of calcium alginate fibres.

11. A process for producing a wound dressing as claimed in claim 1, characterised in that a sheet of polymer foam is coated on one face with a pressure-sensitive adhesive so as to provide a water-vapour permeability of at least 300 grams per square metre per 24 hours at 40°C/80 per cent relative humidity, the adhesive-coated face is optionally covered with a sheet of release material, a frame is cut from the adhesive-coated sheet and a waterproof polymer film having a water-vapour permeability of at least 300 grams per square metre per 24 hours at 40°C/80 per cent relative humidity is secured within the frame by bonding to the pressure-sensitive adhesive after removal of any release material sheet.

FIG.1

FIG.2

FIG.3